# EUROPÄISCHE PATENTANMELDUNG

(11) **EP 3 831 344 A1**
(43) Veröffentlichungstag der Anmeldung: **09.06.2021**
(21) Anmeldenummer: 19213729.7
(22) Anmeldetag: 05.12.2019
(51) Int. Cl.: A61F 2/30, A61B 17/88, A61F 2/46

(54) **SIEGEL ZUR APPLIKATION VON KNOCHENZEMENT**

(71) Anmelder: Heraeus Medical GmbH, 61273 Wehrheim (DE)
(72) Erfinder: SPEHL, Anna, 61273 Wehrheim (DE); WÜST, Edgar, 61273 Wehrheim (DE)
(74) Vertreter: Heraeus IP

(57) **Zusammenfassung**

Die Erfindung betrifft ein Siegel (100) zum Abdichten einer Knochenkanalöffnung beim Applizieren von Knochenzement in die Spongiosa eines Knochenkanals. Die Erfindung betrifft weiterhin eine Vorrichtung (200) zum Applizieren von Knochenzement in die Spongiosa eines Knochenkanals aufweisend das erfindungsgemäße Siegel und ein Verfahren (600) zum Applizieren von Knochenzement mittels der erfindungsgemäßen Vorrichtung.

## Beschreibung

Die Erfindung betrifft ein Siegel zum Abdichten einer Knochenkanalöffnung beim Applizieren von Knochenzement in die Spongiosa eines Knochenkanals. Die Erfindung betrifft weiterhin eine Vorrichtung zum Applizieren von Knochenzement in die Spongiosa eines Knochenkanals aufweisend das erfindungsgemäße Siegel und ein Verfahren zum Applizieren von Knochenzement mittels der erfindungsgemäßen Vorrichtung.

### Hintergrund der Erfindung

Die vollständige Arthroplastik ist ein weithin angewandtes Verfahren in der Orthopädie. Dabei wird infiziertes Gewebe, insbesondere Knochengewebe, entfernt und durch künstliche Prothesen aus Metall oder Kunststoff ersetzt. Üblicherweise werden die Prothesen mittels Knochenzement am verbleibenden, gesunden Knochengewebe fixiert. Insbesondere bei der Fixierung von Hüftprothesen im dafür vorbereiteten medullären Knochenkanal des Femurs besteht, durch die hohe mechanische Beanspruchung, das Risiko einer Lockerung der Prothese, üblicherweise im Bereich des Übergangs von Knochenzement zu Knochen. Um eine verbesserte Haftung zwischen Knochenzement und Knochen zu erreichen, wird der Knochenzement unter erhöhtem Applizierdruck in den Knochenkanal appliziert. Dies führt zu einem, zumindest teilweisen, Eindringen des Knochenzements in die Spongiosa des Knochens, welches aufgrund der hohen Viskosität des Knochenzements bei zu geringem Applizierdruck nicht erfolgen würde, und damit zu einer verbesserten Anbindung von Knochenzement zu Knochen.

Erreicht wird der erhöhte Applizierdruck durch die Verwendung einer mit einem Siegel ausgestatteten Appliziervorrichtung für Knochenzement. Der Knochenzement wird aus der Appliziervorrichtung in den Knochenkanal appliziert, wobei das Siegel während des Appliziervorgangs den Knochenkanal zur Umgebungsatmosphäre derart abdichtet, dass das Applizieren unter ausreichendem Applizierdruck stattfindet um ein Eindringen des Knochenzements in die Spongiosa des Knochens zu bewirken.

Es werden Bemühungen unternommen, Vorrichtungen bereitzustellen, mittels derer Knochenzement unter ausreichendem Applizierdruck in einen Knochenkanal zu applizieren um ein Eindringen des Knochenzements in die Spongiosa des Knochens zu bewirken. Derartige Vorrichtungen, insbesondere Siegel für derartige Vorrichtungen, sind beispielsweise in der US 4,815,454 A und der US 6,017,350 A beschrieben.

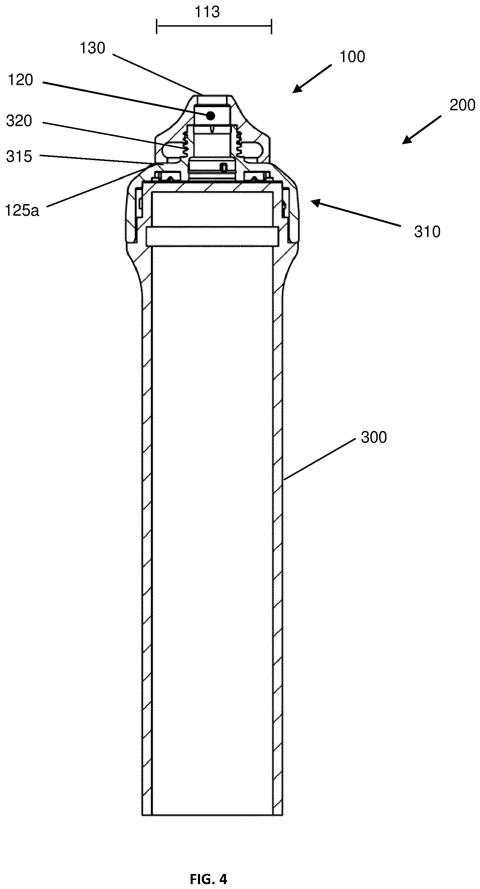

Die US 4,338,925 A ist in erster Linie auf eine Appliziervorrichtung und ein Druckapplizierverfahren von Knochenzement gerichtet, bei dem eine Vielzahl verschiedener Aufsätze für die Appliziervorrichtung verwendet werden. Unter anderem wird ein Siegel, bestehend aus einem Adapter und einem Druckhalter, beschrieben. Der Adapter besteht aus Polyethylen, wird am Ende der Appliziervorrrichtung angeschraubt und weist eine rohrförmige Verlängerung auf. Der Druckhalter besteht aus Silikon und ist konisch (vgl. FIG. 4) geformt. Ein Nachteil dieses Siegels ist, dass es, um den Knochenkanal für die Druckapplizierung abzudichten, tief in den Knochenkanal eindringen muss. Insbesondere bei Revisionen, wenn der Knochenkanal aufgrund der Entfernung von großen Mengen an Knochengewebe zusätzlich geweitet ist, ist die Eindringtiefe des Siegels nochmals deutlich erhöht. Eine zu große Eindringtiefe des Siegels hat mehrere Nachteile. Zum einen kann der Knochenkanal nicht vollständig mit Knochenzement gefüllt werden, sondern der der Appliziervorrichtung zugewandte Bereich bleibt zumindest teilweise ungefüllt und muss in einem weiteren Verfahrensschritt, diesmal ohne die Verwendung des Siegels, aufgefüllt werden. Des Weiteren ist genau dieser, der Appliziervorrichtung zugewandte Bereichs des Knochenkanals, entscheidend für eine erfolgreiche Druckapplizierung des Knochenzements. In diesem Bereich weist der Knochen die sogenannte Spongiosa, eine schwammartige Knochenstruktur, auf, welche das eigentliche Ziel der Druckapplizierung ist. Die Druckapplizierung dient dem Befüllen der Spongiosa, und der daraus resultierenden Verbesserung der Haftung zwischen Knochenzement und Knochen, welche unter einem nicht ausreichendem Applizierdruck aufgrund der hohen Viskosität des Knochenzement nicht zugänglich ist. Sind Bereiche der Spongiosa während des Druckapplizierverfahrens nicht für den Knochenzement zugänglich, verringert dies die Anhaftung von Knochenzement zu Knochen und damit auch die mechanische Stabilität der mit dem Knochenzement zu fixierenden Prothese.
Ein weiterer Nachteil ist die erhöhte Abbruchgefahr des Siegels von der Appliziervorrichtung bei Verkantungen innerhalb des Knochenkanals. Die Druckapplizierung erfordert vom Anwender das Aufbringen eines großen Anpressdruck, mit einer daraus resultierenden großen mechanischen Belastung, des Siegels an den Knochenkanal, welcher aus sterischen Gründen im Verlauf der Operation nicht immer leicht zugänglich ist, was die Abbruchgefahr des Siegels erhöht. Zusätzlich besteht, durch das Vorhandensein verschiedener Körperflüssigkeiten, wie beispielsweise Blut, die Gefahr eines ungewollten Abrutschens des Siegels vom Knochenkanal, was die Abbruchgefahr weiter erhöht.

### Aufgaben

Eine Aufgabe der vorliegenden Erfindung ist es, einen oder mehrere der sich aus dem Stand der Technik ergebenden Nachteile zumindest teilwiese zu überwinden.

Im Speziellen basiert die Erfindung auf dem Ziel, ein Siegel bereitzustellen, welches zumindest eine teilweise Befüllung der Spongiosa eines Knochens mit Knochenzement erlaubt. Das Siegel soll eine im Wesentlichen vollständige Befüllung eines Knochenkanals in nur einem Befüllschritt, ermöglichen. Das Siegel soll eine hohe mechanische Belastbarkeit aufweisen. Das Siegel soll eine geringe Abbruchgefahr bei Verkantungen aufweisen.

Eine weitere Aufgabe der Erfindung ist es, eine Vorrichtung zum Applizieren von Knochenzement in die Spongiosa eines Knochenkanals, mittels derer mindestens ein Teil der bereits beschriebenen Aufgaben zumindest zum Teil gelöst ist.

Es ist eine weitere Aufgabe der Erfindung, ein Verfahren bereitzustellen, mittels dem mindestens ein Teil der bereits beschriebenen Aufgabe zumindest zum Teil gelöst ist.

### Bevorzugte Ausführungsformen der Erfindung

Ein Beitrag zur mindestens teilweisen Erfüllung mindestens einer der zuvor genannten Aufgaben wird durch die Merkmale der unabhängigen Ansprüche geleistet. Die abhängigen Ansprüche stellen bevorzugte Ausführungsformen bereit, die zur mindestens teilweisen Erfüllung mindestens einer der Aufgaben beitragen.
|1| Siegel zum Abdichten einer Knochenkanalöffnung beim Applizieren von Knochenzement in die Spongiosa eines Knochenkanals,
   **dadurch gekennzeichnet, dass**
   das Siegel eine konkave Abdichtungsfläche zum Abdichten des Knochenkanals aufweist.
|2| Siegel nach Ausführungsform 1, **dadurch gekennzeichnet, dass** das Siegel einen Siegelkanal aufweist, wobei der Siegelkanal reversibel form- und/oder kraftschlüssig mit einer Austragsöffnung einer den Knochenzement bereitstellenden Vorrichtung verbindbar ist, um den Knochenzement aus einer Siegelöffnung an einem der Abdichtungsfläche zugewandten Ende des Siegelkanals aus der Vorrichtung in den Knochenkanal zu applizieren.
|3| Siegel nach Ausführungsform 1 oder 2, **dadurch gekennzeichnet, dass** die Siegelöffnung mittig in der Abdichtungsfläche angeordnet ist.
|4| Siegel nach Ausführungsform 2 oder 3, **dadurch gekennzeichnet, dass** die Abdichtungsfläche eine Bauhöhe aufweist, wobei eine maximale Bauhöhe angrenzend an einen Siegelöffnungsbereich vorliegt und die Bauhöhe vom Siegelöffnungsbereich in Richtung der Ränder der Abdichtungsfläche mit einer konkaven Funktion abnimmt.
|5| Siegel nach Ausführungsform 4, **dadurch gekennzeichnet, dass** die konkave Funktion angrenzend an den Siegelöffnungsbereichs eine größte Steigung aufweist und die Steigung in Richtung der Ränder der Abdichtungsfläche stetig abnimmt.
|6| Siegel nach Ausführungsform 4 oder 5, **dadurch gekennzeichnet, dass** die maximale Bauhöhe der Abdichtungsfläche im Bereich von 5 bis 20 mm, bevorzugt von 8 bis 17 mm, weiter bevorzugt von 10 bis 15 mm, liegt.
|7| Siegel nach einer der vorhergehenden Ausführungsformen, **dadurch gekennzeichnet, dass** die Abdichtungsfläche eine ovale Querschnittsfläche mit einer Länge im Bereich von 60 bis 80 mm und einer Breite im Bereich von 25 bis 45 mm aufweist.
|8| Siegel nach einer der vorhergehenden Ausführungsformen, **dadurch gekennzeichnet, dass** das Siegel einen Kunststoff, insbesondere einen thermoplastischen Kunststoff, mit einer Shore-Härte von 25 bis 50 ShoreA umfasst, bevorzugt aus einem Kunststoff, insbesondere aus einem thermoplastischen Kunststoff, mit einer Shore-Härte von 25 bis 50 ShoreA besteht.
|9| Vorrichtung zum Applizieren von Knochenzement in die Spongiosa eines Knochenkanals, umfassend
   ein Behältnis, in dem ein Knochenzement lagerbar ist,
   wobei das Behältnis an einem Ende eine Austragsseite mit einer aus der Austragsseite herausragenden Austragsöffnung zum Austragen des Knochenzements aus dem Behältnis in den Knochenkanal aufweist,
   **dadurch gekennzeichnet, dass**
   die Austragsöffnung reversibel form- und/oder kraftschlüssig mit einem Siegel nach einer der Ausführungsformen 1 bis 8 zum Abdichten einer Knochenkanalöffnung verbunden ist, um den Knochenzement unter ausreichendem Applizierdruck in den Knochenkanal zu applizieren.
|10| Vorrichtung nach Ausführungsform 9, **dadurch gekennzeichnet, dass** das Siegel wenigstens partiell formschlüssig an einer Austragsseitenaußenfläche angeordnet ist
|11| Vorrichtung zum Applizieren von Knochenzement in die Spongiosa eines Knochenkanals, umfassend
   ein Behältnis, in dem ein Knochenzement lagerbar ist,
   wobei das Behältnis an einem Ende eine Austragsseite mit einer aus der Austragsseite herausragenden Austragsöffnung zum Austragen des Knochenzements aus dem Behältnis in den Knochenkanal aufweist,
   ein Siegel zum Abdichten einer Knochenkanalöffnung, um den Knochenzement unter ausreichendem Applizierdruck in den Knochenkanal zu applizieren,
   wobei das Siegel reversibel form- und/oder kraftschlüssig mit der Austragsöffnung verbunden ist,
   **dadurch gekennzeichnet, dass**
   das Siegel wenigstens partiell formschlüssig an einer Austragsseitenaußenfläche angeordnet ist.
|12| Vorrichtung nach Ausführungsform 9 bis 11, **dadurch gekennzeichnet, dass** der Applizierdruck ausreicht, um den Knochenzement mindestens 2 mm, insbesondere zwischen 3 bis 5 mm, tief in die Spongiosa eindringen zu lassen.
|13| Vorrichtung nach einer der Ausführungsformen 9 bis 12, **dadurch gekennzeichnet, dass** das Siegel eine der Abdichtungsfläche gegenüberliegende Rückseitenfläche aufweist, wobei die Rückseitenfläche formschlüssig an der Austragsseitenaußenfläche angeordnet ist.
|14| Vorrichtung nach Ausführungsform 13, **dadurch gekennzeichnet, dass** die Rückseitenfläche mit zumindest 20 Flächen-% an der Austragsseitenaußenfläche angeordnet ist.
|15| Vorrichtung nach Ausführungsform 13 oder 14, **dadurch gekennzeichnet, dass** die Rückseitenfläche kreisringförmig an der Austragsseitenaußenfläche angeordnet ist.
|16| Verfahren zum Applizieren von Knochenzement in die Spongiosa eines Knochenkanals mittels einer Vorrichtung nach einer der Ausführungsformen 9 bis 15, zumindest umfassend die folgenden Schritte:
   a) Bereitstellen des Knochenzements innerhalb der Vorrichtung,
   b) Anpressen der Vorrichtung auf eine Knochenkanalöffnung, so dass der Knochenkanal während des Applizierens des Knochenzements durch ein Siegel abgedichtet ist,
   c) Austragen des Knochenzements aus der Vorrichtung in den Knochenkanal,
   wobei das Siegel im Wesentlichen auf eine Oberfläche des Knochens und/oder auf eine Schnittfläche des Knochens gepresst wird.
|17| Verfahren nach Ausführungsform 16, **dadurch gekennzeichnet, dass** in Schritt c) der Knochenkanal im Wesentlichen vollständig mit Knochenzement gefüllt wird.

### Allgemeines

In der vorliegenden Beschreibung beinhalten Bereichsangaben auch die als Grenzen genannten Werte. Eine Angabe der Art "im Bereich von X bis Y" in Bezug auf eine Größe A bedeutet folglich, dass A die Werte X, Y und Werte zwischen X und Y annehmen kann. Einseitig begrenzte Bereiche der Art "bis zu Y" für eine Größe A bedeuten entsprechend als Wert Y und kleiner als Y.

Einige der beschriebenen Merkmale sind mit dem Begriff "im Wesentlichen" verknüpft. Der Begriff "im Wesentlichen" ist so zu verstehen, dass unter realen Bedingungen und Fertigungstechniken eine mathematisch exakte Auslegung von Begrifflichkeiten wie "Überlagerung", "senkrecht", "Durchmesser" oder "Parallelität" nie exakt, sondern nur innerhalb gewisser fertigungstechnischer Fehlertoleranzen gegeben sein kann. Beispielsweise schließen "im Wesentlichen parallele Achsen" einen Winkel von 85 Grad bis 95 Grad zueinander ein und "im Wesentlichen gleiche Volumen" umfassen eine Abweichung von bis zu 5 Volumen-%. Eine "im Wesentlichen aus Kunststoff bestehende Vorrichtung" umfasst beispielsweise einen Kunststoffanteil von ≥95 bis ≤100 Gewichts-%. Eine "im Wesentlichen vollständige Befüllung eines Volumens B" umfasst beispielsweise eine Befüllung von ≥95 bis ≤100 Volumen-% des Gesamtvolumens von B.

### Ausführliche Beschreibung

Ein erster Gegenstand der Erfindung betrifft ein Siegel zum Abdichten einer Knochenkanalöffnung beim Applizieren von Knochenzement in die Spongiosa eines Knochenkanals, dadurch gekennzeichnet, dass das Siegel eine konkave Abdichtungsfläche zum Abdichten des Knochenkanals aufweist.

Ein Siegel ist ein fluidleitendes Element, welches reversibel an eine Vorrichtung angebracht werden kann, um einen Knochenzement aus der Vorrichtung unter derart hohem Applikationsdruck in einen Knochenkanal zu applizieren, dass der Knochenzement zumindest teilweise in die Spongiosa des Knochenkanals eingebracht wird. Dabei verbindet das Siegel die Vorrichtung und den Knochenkanal fluidleitend, um ein Applizieren des Knochenzements aus der Vorrichtung in den Knochenkanal zu ermöglichen, verhindert aber gleichzeitig ein Austreten des Knochenzements aus der Knochenkanalöffnung. Mit fortgesetztem Applizieren des Knochenzements in den Knochenkanal kommt es dadurch zu einem ausreichenden hohen Applizierdruck, welcher zumindest ein teilweises Einbringen des Knochenzements in die Spongiosa des Knochenkanals bewirkt. Aufgrund der teigähnlichen, hochviskosen Beschaffenheit des Knochenzements bedarf es, in Abhängigkeit der konkreten Knochenzementbeschaffenheit, einen Mindestbetrag an Applizierdruck. Wird dieser Mindestbetrag unterschritten, findet kein Einbringen des Knochenzements in die Spongiosa statt. Der Mindestbetrag des Applizierdrucks entspricht einem ausreichenden Applizierdruck. Der für die Arthoplastik verwendete Knochenzement weist beispielsweise 2 bis 6 Minuten nach dem Anmischen eine dynamische Viskosität bei Raumtemperatur (ca. 20 bis 22 °C) im Bereich von 100 bis 500 Pa*s auf.

Unter einem ausreichendem Applizierdruck wird verstanden, dass ein fortgeführtes Fördern des Knochenzements in den Knochenkanal bei mit dem Siegel abgedichteter Knochenkanalöffnung zu einem Einbringen des Knochenzements in die Spongiosa des Knochens um mindestens 2 mm, insbesondere zwischen 3 und 5 mm, möglich ist.

Ein zumindest teilweises Einbringen des Knochenzements in die Spongiosa eines Knochenkanals, insbesondere eines Femurknochenkanals, sorgt für eine erhöhte Haftkraft zwischen dem entsprechenden Knochen und dem Knochenzement und damit zu einer verbesserten Fixierung einer mit dem Knochenzement fixierten Prothese, insbesondere einer Hüftprothese.

Zum Applizieren wird die mit dem Siegel ausgestattete Vorrichtung derart gegen den Knochenkanal gepresst, dass eine dem Knochenkanal zugewandte Abdichtungsfläche des Siegels den Knochenkanal abdichtet und ein Applizieren des Knochenzements aus der Vorrichtung in den Knochenkanal unter ausreichendem Applizierdruck ermöglicht.

Die Abdichtungsfläche des Siegels ist konkav ausgestaltet. Eine konkave Abdichtungsfläche erlaubt einerseits eine zuverlässige Abdichtung des Knochenkanals und zum anderen eine im Wesentlichen vollständige Befüllung des Knochenkanals mit Knochenzement unter ausreichendem Applizierdruck, insbesondere eine vollständige Befüllung des Knochenkanals mit Knochenzement in einem einzigen Applizierschritt. Durch die konkave Ausgestaltung ist die Abdichtungsfläche während des Applizierens des Knochenzements im Wesentlichen nicht in Kontakt mit der Spongiosa, sondern davon beabstandet, was einen freien Zugang des Knochenzements zur Spongiosa und damit ein Einbringen desselben in die Spongiosa ermöglicht.

Die konkave Abdichtungsfläche kann auf unterschiedlichen Arten ausgestaltet sein.

In einer Ausgestaltungsform ist die Abdichtungsfläche als Kugelschale, beispielsweise als Halbkugelschale, ausgeformt, wobei die Kugelschale einen kreisförmig, elliptisch oder unregelmäßig geformten Querschnitt aufweisen kann. Dabei wird die kreisringförmige Außenfläche der Kugelschale gegen den Knochen gepresst, so dass der Knochenkanal gegen die Umgebungsatmosphäre abgedichtet ist, während der Knochenzement durch eine entsprechende Öffnung der Kugelschale in den Knochenkanal appliziert wird. Etwaig überschüssiger Knochenzement, welcher nach dem Applizieren in dem kugelschalenförmigen Siegel verbleibt, kann durch den Operateur nach Befüllen des Knochenkanals, beispielsweise per Hand, entfernt werden.

Eine weitere Ausgestaltungsform des Siegels ist dadurch gekennzeichnet, dass das Siegel einen Siegelkanal aufweist, wobei der Siegelkanal reversibel form- und/oder kraftschlüssig mit einer Austragsöffnung einer den Knochenzement bereitstellenden Vorrichtung verbindbar ist, um den Knochenzement aus einer Siegelöffnung an einem der Abdichtungsfläche zugewandten Ende des Siegelkanals aus der Vorrichtung in den Knochenkanal zu applizieren.

Das Siegel weist einen Siegelkanal auf. Unter einem Siegelkanal ist eine fluidleitende, rohrartige Verbindung in axialer Erstreckung des Siegels zu verstehen, welche es erlaubt, den Knochenzement von einer der Vorrichtung zugwandten Seite in Richtung der dem Knochenkanal zugewandten Abdichtungsfläche des Siegels zu fördern. Der Siegelkanal mündet auf Seite der Abdichtungsfläche in die Siegelöffnung.

Zum Fördern des Knochenzements ist der Siegelkanal form- und/oder kraftschlüssig mit der Austragsöffnung der Vorrichtung verbunden. In einer ersten Ausgestaltungsform weist der Siegelkanal ein Innengewinde auf, welches mit einem Außengewinde der Austragsöffnung zusammenwirkt, um den Knochenkanal, und damit auch das Siegel, mit der Austragsöffnung zu verbinden. In einer weiteren Ausgestaltungsform weist der Siegelkanal zumindest bereichsweise einen Innenquerschnitt auf, welcher im Wesentlichen einem Außendurchmesser der Austragsöffnung entspricht, um so den Siegelkanal über einen Form- und/oder Kraftschluss mit der Austragsöffnung zu verbinden. In einer weiteren Ausgestaltungsform weist das Siegel ein Verbindungselement, beispielsweise ein Gewinde oder einen Bajonettverschluss, auf, welche das Siegel mit der Vorrichtung verbindet, so dass der Siegelkanal im Wesentlichen formschlüssig an die Austragsöffnung anliegt und ein Fördern des Knochenzements durch den Siegelkanal ermöglicht. In einer weiteren Ausgestaltung ist der Querschnitt des Siegelkanals über die gesamte axiale Erstreckung des Siegelkanals gleich groß. In einer weiteren Ausgestaltungsform weist der Siegelkanal eine konische Form aus, wobei vorzugsweise der Querschnitt des Siegelkanals in Richtung der Siegelöffnung größer wird.

Der Siegelkanal kann beliebige axiale Verläufe durch das Siegel aufweisen. In einer bevorzugten Ausgestaltungform weist der Siegelkanal einen im Wesentlichen geradlinigen axialen Verlauf durch das Siegel auf.

Die Siegelöffnung kann an unterschiedlichen Positionen der Abdichtungsfläche angebracht sein.

Eine weitere Ausgestaltungform des Siegels ist dadurch gekennzeichnet, dass die Siegelöffnung mittig in der Abdichtungsfläche angeordnet ist.

Unter einer mittig angebrachten Siegelöffnung ist zu verstehen, dass sich die Siegelöffnung im Bereich des Flächenschwerpunkts einer Projektion der Abdichtungsfläche, und nicht im Randbereich der Abdichtungsfläche, befindet. Bei einer rotationssymmetrischen Ausgestaltung der Abdichtungsfläche entspräche dies dem Bereich der Rotationsachse.

Eine mittige Anordnung der Siegelöffnung erleichtert dem Operateur die Verwendung des Siegels, da eine Drehung der Vorrichtung um eine Längsachse während des Applizierens, oder zumindest des Abdichtens des Knochenkanals, keine oder nur eine geringe räumliche Verschiebung der Siegelöffnung gegen den Knochenkanal nach sich zieht.

Eine weitere Ausgestaltungsform des Siegels ist dadurch gekennzeichnet, dass die Abdichtungsfläche eine Bauhöhe aufweist, wobei eine maximale Bauhöhe angrenzend an einen Siegelöffnungsbereich vorliegt und die Bauhöhe vom Siegelöffnungsbereich in Richtung der Ränder der Abdichtungsfläche mit einer konkaven Funktion abnimmt.

Unter einer Bauhöhe ist eine axiale Erstreckung der Abdichtungsfläche entlang einer Längsachse des Siegels zu verstehen, wobei die Ränder, oder zumindest der der Vorrichtung in axialer Erstreckung am nächsten kommende Rand, eine zu der Längsachse des Siegels senkrecht liegende Ebene definieren, gegen welche die Bauhöhe bestimmt wird.

Das Siegel weist einen Siegelöffnungsbereich auf. Ein Siegelöffnungsbereich grenzt radial direkt an die Siegelöffnung und umgibt die Siegelöffnung zumindest abschnittsweise, bevorzugt vollständig, in radialer Erstreckung. Der Siegelöffnungsbereich erstreckt sich im Wesentlichen senkrecht zu einer Längsachse des Siegels und geht in die Abdichtungsfläche über. Der Siegelöffnungsbereich weist eine radiale Erstreckung um die Siegelöffnung in einem Bereich von 1 mm bis 2 cm auf.

Ein Vorteil dieser Ausgestaltungform ist, dass dadurch der Bereich der Siegelöffnung während Abdichtens und Applizieren des Knochenzements, wenn auch aufgrund der konkaven Form der Abdichtungsfläche nur gering, in den Knochenkanal eingeführt wird. Dies erleichtert dem Operateur zum einen ein passgenaues Anpressen des Siegels an den Knochenkanal durch eine haptische Rückmeldung während des Einbringens der Siegelöffnung in den Knochenkanal. Des Weiteren verringert sich auf diese Weise die Gefahr eines unbeabsichtigten Abrutschens des Siegels aus der Öffnung des Knochenkanals während des Applizierens, da die Bauhöhe der Abdichtungsfläche das Abrutschen erschwert.

Durch die Abnahme der Bauhöhe der Abdichtungsfläche mittels konkaver Funktion wird ein freier Zugang des Knochenzements in die Spongiosa der Knochenkanals im Bereich der Öffnung des Knochenkanals und gleichzeitig eine geringe Eindringtiefe der Siegelöffnung in den Knochenkanal gewährleistet. Gleichzeitig erlaubt die Form des Siegels dem Operateur ein sicheres und stabiles Anpressen der Vorrichtung an den Knochenkanal, auch unter großen Anpressdruck. Zudem kann der Knochenkanal in einem Verfahrensschritt, ohne Absetzen der Vorrichtung und entfernen des Siegels, im Wesentlichen vollständig mit Knochenzement befüllt werden.

Damit der freie Zugang des Knochenzements zur Spongiosa des Knochens noch weiter erleichtert wird und zugleich die Eindringtiefe der Siegelöffnung möglichst gering ausfällt, ist eine weitere Ausgestaltungsform des Siegels dadurch gekennzeichnet, dass die konkave Funktion angrenzend an den Siegelöffnungsbereich die größte Steigung aufweist und die Steigung in Richtung der Ränder der Abdichtungsfläche stetig abnimmt.

In einer Ausgestaltungsform weist die konkave Funktion direkt angrenzend an den Siegelöffnungsbereich die größte Steigung auf. In weiteren Ausgestaltungsformen weist die konkave Funktion nicht direkt angrenzend an den Siegelöffnungsbereich die größte Steigung auf, sondern die größte Steigung ist vom Siegelöffnungsbereich, insbesondere von einem Rand des Siegelöffnungsbereich, bis zu 5 mm, bevorzugt bis zu 3 mm, weiter bevorzugt bis zu 1 mm, beabstandet. Unabhängig, ob die konkave Funktion direkt angrenzend an den Siegelöffnungsbereich oder beabstandet zum Siegelöffnungsbereich, insbesondere beabstandet von einem Rand der Siegelöffnungsbereich, ist, nimmt die Steigung der konkaven Fläche vom Punkt der maximalen Steigung in Richtung der Ränder der Abdichtungsfläche stetig ab.

Eine weitere Ausführungsform des Siegels ist dadurch gekennzeichnet, dass die maximale Bauhöhe der Abdichtungsfläche im Bereich von 5 bis 20 mm, bevorzugt von 8 bis 17 mm, weiter bevorzugt von 10 bis 15 mm, liegt.

Ein Vorteil der maximalen Bauhöhe im Bereich von 5 bis 20 mm, bevorzugt von 8 bis 17 mm, weiter bevorzugt von 10 bis 15 mm ist ein ausgewogener Kompromiss zwischen geringer Eindringtiefe des Siegels in den Knochenkanal bei gleichzeitig guter Haftung beim Anpressen der Vorrichtung auf den Knochenkanal.

Die Abdichtungsfläche kann beliebige Querschnittsflächenformen aufweisen. Beispielsweise können die Querschnittsflächen rund ausgestaltet sein.

Eine weitere Ausführungsform des Siegels ist dadurch gekennzeichnet, dass die Abdichtungsfläche eine ovale Querschnittsfläche mit einer Länge im Bereich von 60 bis 80 mm und einer Breite im Bereich von 25 bis 45 mm aufweist.

Ovale, insbesondere eiförmig ovale, Querschnittsflächen sind aufgrund einer guten formlichen Übereinstimmung mit den typischerweise im Querschnitt ovalen Knochenkanälen und aufgrund einer guten Anpassbarkeit an verschieden große Knochenkanalöffnungen bevorzugt. Zudem kann, durch eine Drehung um die Längsachse des Siegels, eine Anlagefläche der Abdichtungsfläche an den Knochen gut an die bestehende Form der Knochenkanalöffnung angepasst werden. Eine Länge im Bereich von 60 bis 80 mm und eine Breite von 25 bis 45 mm erlaubt eine Verwendung des Siegels für eine Vielzahl unterschiedlicher Öffnungsquerschnitte von Knochenkanälen, insbesondere für Knochenkanäle, welche im Zuge einer Revision eine vergrößerte Knochenkanalöffnung aufweisen.

Das Siegel kann aus unterschiedlichen Materialien gefertigt sein. Um eine gute Abdichtung des Knochenkanals, insbesondere bei Knochenkanalöffnungen, welche aus sterischen Gründen nur aus einem ungünstigen, nicht axialen Anpresswinkel zugänglich sind oder eine unregelmäßige Knochenoberfläche im Bereich der Knochenkanalöffnung besitzen, ist es bevorzugt, dass das Siegel aus einem verformbaren Material besteht.

Eine weitere Ausgestaltungsform des Siegels ist dadurch gekennzeichnet, dass das Siegel einen Kunststoff, insbesondere einen thermoplastischen Kunststoff, mit einer Shore-Härte von 25 bis 50 ShoreA umfasst, bevorzugt aus einem Kunststoff, insbesondere aus einem thermoplastischen Kunststoff, mit einer Shore-Härte von 25 bis 50 ShoreA besteht.

Eine größere Shore-Härte als 50 ShoreA des Kunstoffs resultiert, insbesondere bei nicht axialem Anpresswinkel an die Knochenkanalöffnung, aufgrund eines nicht vorhandenen Formschlusses zwischen Siegel und Knochen in einer nicht ausreichenden Abdichtung des Knochenkanals, so dass ein ausreichender Applizierdruck für den Knochenzement nicht erreicht werden kann.

Ist auf der anderen Seite die Shore-Härte kleiner als 25 ShoreA, ist der Kunststoff zu weich, so dass das Siegel zwar einen Formschluss mit der Knochenkanalöffnung ermöglicht, aber gleichzeitig der Knochenkanal aufgrund der zu hohen Flexibilität des Siegels nicht ausreichend abgedichtet wird. Ein erhöhter Applizierdruck presst bei zu weichem Kunststoff den Knochenzement, anstatt in die Spongiosa hinein, an dem Siegel vorbei aus dem Knochenkanal heraus.

Beispiele für solche Kunststoffe umfassen Kunststoffe aus den Kunststoffklassen der Silikone, der thermoplastischen Elastomere (TPE) und/oder der thermoplastischen Vulkanisate (TPV), wie beispielsweise Santopren.

Ein weiterer Gegenstand der Erfindung betrifft eine Vorrichtung zum Applizieren von Knochenzement in die Spongiosa eines Knochenkanals, umfassend ein Behältnis, in dem ein Knochenzement lagerbar ist, wobei das Behältnis an einem Ende eine Austragsseite mit einer aus der Austragsseite herausragenden Austragsöffnung zum Austragen des Knochenzements aus dem Behältnis in den Knochenkanal aufweist, dadurch gekennzeichnet, dass die Austragsöffnung reversibel form- und/oder kraftschlüssig mit einem Siegel nach einer der vorhergehenden Ausgestaltungsformen zum Abdichten einer Knochenkanalöffnung verbunden ist, um den Knochenzement unter ausreichendem Applizierdruck in den Knochenkanal zu applizieren.

Die Vorrichtung weist ein Behältnis auf. Unter einem Behältnis ist ein rohrartiges Gefäß, welches einen Innenraum und eine den Innenraum umgebende Behältniswand umfasst, zu verstehen. Das Behältnis besitzt senkrecht zu einer Längsachse einen Querschnitt. Erfindungsgemäß kann der Querschnitt des Behältnisses beliebige Formen annehmen. Beispielsweise kann der Querschnitt oval, quadratisch, fünfeckig, sechseckig, unregelmäßig oder kreisförmig ausgestaltet sein.

Es ist bevorzugt, dass das Behältnis eine zylindrische Geometrie mit rotationssymmetrischer Achse mit rundem Querschnitt aufweist. Diese Geometrie erlaubt eine gute Handhabbarkeit für den Anwender und reduziert durch eine Abwesenheit von Kanten ein Risiko einer Verkeilung bei der Verwendung der Vorrichtung. Erfindungsgemäß kann das Behältnis aus unterschiedlichsten Materialien oder Materialkombinationen bestehen. Beispiele kann das Behältnis aus Kunststoff bestehen. Bevorzugt handelt es sich bei dem Kunststoff um einen transparenten Kunststoff, da der Anwender auf diese Weise eine ordnungsgemäße Funktion der Vorrichtung während einer Verwendung optisch kontrollieren kann.

Das Behältnis weist an einem Ende, insbesondere einem axialen Ende, eine Austragsseite auf, welche das Behältnis abschließt. Die Austragsseite umfasst eine Austragsöffnung, durch welche der Knochenzement aus dem Innenraum des Behältnisses gefördert werden kann, wobei sich die Austragsöffnung axial in Richtung der dem Innenraum des Behältnisses abgewandten Seite der Austragsseite erstreckt. Die herausragende Austragsöffnung erlaubt eine reversible Befestigung von Hilfsmitteln, wie beispielsweise einem Schnorchel oder einem Siegel, welche bei der Applikation des Knochenzements nützlich oder notwendig sind.

Die Befestigung des Siegels kann auf unterschiedliche Weisen erfolgen. In einer Ausgestaltungsform weist die Austragsöffnung ein Innengewinde auf, welches mit einem Außengewinde des Siegels zusammenwirkt, um eine form- und/oder kraftschlüssige Verbindung herzustellen. In einer weiteren Ausgestaltungsform weist die Austragsöffnung ein Außengewinde auf, welches mit einem Innengewinde des Siegels zusammenwirkt, um eine form- und/oder kraftschlüssige Verbindung herzustellen. In einer weiteren Ausgestaltungsform werden Austragsöffnung und Siegel über eine Bajonettverbindung miteinander verbunden. In einer weiteren Ausgestaltungsform weist das Siegel eine Ausnehmung auf, welche form- und/oder kraftschlüssig mit der aus der Austragsseite herausragenden Austragsöffnung zusammenwirkt, um das Siegel zu befestigen. In einer weiteren, bevorzugten, Ausgestaltungsform entspricht der Innendurchmesser des Siegelkanals im Wesentlichen dem Außendurchmesser der Austragsöffnung, wodurch das Siegel durch ein Einschieben der Austragsöffnung in den Siegelkanal form- und/oder kraftschlüssig mit der Austragsöffnung verbindbar ist.

In einer Ausgestaltungsform der Vorrichtung ist das verbundene Siegel ausschließlich an der Austragsöffnung angeordnet und nicht in direktem physischem Kontakt mit weiteren Teilen der Vorrichtung.

Eine weitere Ausgestaltungsform der Vorrichtung ist dadurch gekennzeichnet, dass das Siegel wenigstens partiell formschlüssig an einer Austragsseitenaußenfläche angeordnet ist.

Die Austragsseitenaußenfläche ist die dem Siegel zugewandte und die dem Innenraum des Behältnisses abgewandte Seite der Austragsseite.

Unter einer formschlüssigen Anordnung ist zu verstehen, dass das Siegel und die Austragsseitenaußenfläche zumindest bereichsweise in direktem physischem Kontakt stehen. In dieser Ausgestaltungsform ist das Siegel sowohl mit der Austragsseitenöffnung, zur Befestigung des Siegels an der Vorrichtung, als auch mit der Austragsseitenaußenfläche in direktem physischem Kontakt.

Ein Vorteil des formschlüssigen Anliegens des Siegels an der Austragsseitenfläche ist eine hohe mechanische Belastbarkeit des Siegels beim Anpressen gegen den Knochenkanal. Das Anliegen an der Austragsseite sorgt für eine flächigere Kraftverteilung auf das Siegel beim Anpressen der Vorrichtung gegen den Knochenkanal, als dies bei nur punktueller Befestigung des Siegels, beispielsweise bei ausschließlichem physischem Kontakt mit der Austragsöffnung, der Fall wäre. Insbesondere wird durch das formschlüssige Anliegen die Verbindung zwischen Siegel und Vorrichtung mechanisch weniger beansprucht. Dadurch erhöht sich nicht nur die mechanische Belastbarkeit des Siegels, sondern auch die mechanische Belastbarkeit der Vorrichtung als Ganzes, insbesondere der Befestigungsstelle des Siegels, wie beispielsweise der Austragsöffnung. Vorteilhaft ist dies insbesondere bei Verkantungen des Siegels im Knochenkanal, welche aufgrund des hohen benötigten Anpressdrucks an den Knochen bei geringer mechanischer Stabilität zu Brüchen der Vorrichtung führen können. Die erhöhte mechanische Stabilität des Siegels und der Vorrichtung als Ganzes, erlaubt dem Operateur das Ausüben eines größeren Anpressdrucks an den Knochenkanal, was insbesondere bei nicht senkrechtem Anpresswinkel der Vorrichtung an den Knochen, ein vollständiges Abdichten der Knochenkanalöffnung erlaubt.

Eine weitere Ausgestaltungsform der Vorrichtung ist dadurch gekennzeichnet, dass der Applizierdruck ausreicht, um den Knochenzement mindestens 2 mm, insbesondere zwischen 3 bis 5 mm, tief in die Spongiosa eindringen zu lassen.

Eine geringere Eindringtiefe als 2 mm führt dazu, dass sich der Knochenzement, und die mit dem Knochenzement fixierte Prothese, insbesondere Hüftprothese, durch die Beanspruchungen des Gebrauchs, insbesondere bei Gehbewegungen des Patienten, lockert. Dies führt zu Folgeoperationen, welche bei einer Eindringtiefe über 2 mm vermieden, oder zumindest zeitlich herausgezögert werden können.

Sollte sich der Bereich der fixierten Prothese entweder im Anschluss an die Operation oder im Laufe der Zeit infizieren, muss die Prothese ausgewechselt werden. Dies erfolgt, unter anderem, durch die vollständige Entfernung des zur Fixierung verwendeten Knochenzements mitsamt des mit Knochenzements durchdrungenen Bereichs der Spongiosa. Um nicht übermäßig große Teile der Spongiosa zu Entfernen und auch bei der nachfolgenden Fixierung der neuen Prothese noch einen Teil der nicht entfernten Spongiosa wieder zur verbesserten Anhaftung von Knochen zu Knochenzement verwenden zu können, ist es bevorzugt, den Knochenzement nicht mehr als 5 mm tief in die Spongiosa eindringen zu lassen.

Eine weitere Ausgestaltungsform der Vorrichtung ist dadurch gekennzeichnet, dass das Siegel eine der Abdichtungsfläche gegenüberliegende Rückseitenfläche aufweist, wobei die Rückseitenfläche formschlüssig an der Austragsseitenaußenfläche angeordnet ist.

Der formschlüssige Kontakt zwischen der Rückseitenfläche und der Austragsseitenaußenfläche kann unterschiedlich ausgeformt sein. In einer Ausgestaltungsform sind zumindest 10 Flächen-% der Rückseitenfläche formschlüssig an der Austragsseitenaußenfläche angeordnet. Unter Flächen-% der Rückseitenfläche ist der Anteil der Rückseitenfläche zu verstehen, welcher, bezogen auf die Gesamtfläche der Rückseitenfläche, formschlüssig an der Austragsseitenfläche anliegt. Je höher dieser Anteil ist, desto größer ist die mechanische Belastbarkeit des Siegels, und damit auch die der Vorrichtung als Ganzes.

Eine weitere Ausgestaltungsform der Vorrichtung ist dadurch gekennzeichnet, dass die Rückseitenfläche mit zumindest 20 Flächen-% an der Austragsseitenaußenfläche angeordnet ist.

Die Rückseitenfläche kann mit bis zu 100 Flächen-% an der Austragsseitenaußenfläche angeordnet sein.

Die Rückseitenfläche, insbesondere der formschlüssig an die Austragsseitenaußenfläche angeordnete Teil der Rückseitenfläche, kann unterschiedlich ausgeformt sein. In einer Ausgestaltungsform ist die Rückseitenfläche planar ausgestaltet. In einer weiteren Ausgestaltungsform ist die gesamte Rückseitenfläche als Negativform der Austragsseitenaußenfläche ausgestaltet, so dass die gesamte Rückseitenfläche oder zumindest ein Teil der Rückseitenfläche formschlüssig an der Austragsseitenaußenfläche angeordnet ist.

Eine weitere Ausgestaltungsform der Vorrichtung ist dadurch gekennzeichnet, dass die Rückseitenfläche kreisringförmig an der Austragsseitenaußenfläche angeordnet ist.

Dazu weist die Rückseitenfläche eine kreisringförmige Struktur, beispielsweise ausgestaltet als eine Erhebung oder eine Vertiefung, auf, welche zumindest abschnittsweise als Negativform der Austragsseitenaußenfläche ausgeformt ist. Vorzugswiese befindet sich die kreisringförmige Struktur im Bereich der Ränder des Siegels, und damit auch im Bereich der durch die Außenwände des Behältnisses stabilisierten Ränder der Vorrichtung, so dass das Siegel insbesondere im Bereich der Siegelränder formschlüssig an der Vorrichtung anliegt, was eine erhöhte mechanische Belastbarkeit der Vorrichtung, insbesondere bei Verkantungen und nicht senkrechten Anpresswinkel der Vorrichtung an den Knochenkanal, sicherstellt. Das kreisringförmige Anliegen des Siegels an der Austragsseitenaußenfläche führt weiterhin zu einem möglichst breitgefächerten Anliegen über einen weiten Teil des Siegels und erhöht somit die mechanische Belastbarkeit des Siegels als auch der Vorrichtung als Ganzes. Vorzugsweise erstreckt sich die kreisringförmige Struktur der Rückseitenfläche nicht bis in den Bereich der Austragsöffnung, so dass die mechanische Belastung während des Anpressens an den Knochenkanal hauptsächlich auf die stabilen Ränder der Vorrichtung ausgeübt wird und nicht auf die weniger stabile Austragsöffnung.

Ein weiterer Gegenstand der Erfindung betrifft eine Vorrichtung zum Applizieren von Knochenzement in die Spongiosa eines Knochenkanals, umfassend ein Behältnis, in dem ein Knochenzement lagerbar ist, wobei das Behältnis an einem Ende eine Austragsseite mit einer aus der Austragsseite herausragenden Austragsöffnung zum Austragen des Knochenzements aus dem Behältnis in den Knochenkanal aufweist, ein Siegel zum Abdichten einer Knochenkanalöffnung, um den Knochenzement unter ausreichendem Applizierdruck in den Knochenkanal zu applizieren, wobei das Siegel reversibel form- und/oder kraftschlüssig mit der Austragsöffnung verbunden ist, dadurch gekennzeichnet, dass das Siegel wenigstens partiell formschlüssig an einer Austragsseitenaußenfläche angeordnet ist.

Die Vorrichtung weist ein Behältnis auf. Unter einem Behältnis ist ein rohrartiges Gefäß, welches einen Innenraum und eine den Innenraum umgebende Behältniswand umfasst. Das Behältnis besitzt senkrecht zu einer Längsachse einen Querschnitt. Erfindungsgemäß kann der Querschnitt des Behältnisses beliebige Formen annehmen. Beispielsweise kann der Querschnitt oval, quadratisch, fünfeckig, sechseckig, unregelmäßig oder kreisförmig ausgestaltet sein.

Es ist bevorzugt, dass das Behältnis eine zylindrische Geometrie mit rotationssymmetrischer Achse mit rundem Querschnitt aufweist. Diese Geometrie erlaubt eine gute Handhabbarkeit für den Anwender und reduziert durch eine Abwesenheit von Kanten ein Risiko einer Verkeilung bei der Verwendung der Vorrichtung. Erfindungsgemäß kann das Behältnis aus unterschiedlichsten Materialien oder Materialkombinationen bestehen. Beispiele kann das Behältnis aus Kunststoff bestehen. Bevorzugt handelt es sich bei dem Kunststoff um einen transparenten Kunststoff, da der Anwender auf diese Weise eine ordnungsgemäße Funktion der Vorrichtung während einer Verwendung optisch kontrollieren kann.

Das Behältnis weist an einem Ende, insbesondere einem axialen Ende, eine Austragsseite auf, welche das Behältnis abschließt. Die Austragsseite umfasst eine Austragsöffnung, durch welche der Knochenzement aus dem Innenraum des Behältnisses gefördert werden kann, wobei sich die Austragsöffnung axial in Richtung der dem Innenraum des Behältnisses abgewandten Seite der Austragsseite erstreckt. Die herausragende Austragsöffnung erlaubt eine reversible Befestigung von Hilfsmitteln, wie beispielsweise einem Schnorchel oder einem Siegel, welche bei der Applikation des Knochenzements nützlich oder notwendig sind.

Die Befestigung des Siegels kann auf unterschiedliche Weisen erfolgen. In einer Ausgestaltungsform weist die Austragsöffnung ein Innengewinde auf, welches mit einem Außengewinde des Siegels zusammenwirkt, um eine form- und/oder kraftschlüssige Verbindung herzustellen. In einer weiteren Ausgestaltungsform weist die Austragsöffnung ein Außengewinde auf, welches mit einem Innengewinde des Siegels zusammenwirkt, um eine form- und/oder kraftschlüssige Verbindung herzustellen. In einer weiteren Ausgestaltungsform werden Austragsöffnung und Siegel über eine Bajonettverbindung miteinander verbunden. In einer weiteren Ausgestaltungsform weist das Siegel eine Ausnehmung auf, welche form- und/oder kraftschlüssig mit der aus der Austragsseite herausragenden Austragsöffnung zusammenwirkt, um das Siegel zu befestigen. In einer weiteren, bevorzugten, Ausgestaltungsform entspricht der Innendurchmesser des Siegelkanals im Wesentlichen dem Außendurchmesser der Austragsöffnung, wodurch das Siegel durch ein Einschieben der Austragsöffnung in den Siegelkanal form- und/oder kraftschlüssig mit der Austragsöffnung verbindbar ist.

Das Siegel ist wenigstens partiell formschlüssig an einer Austragsseitenaußenfläche angeordnet. Die Austragsseitenaußenfläche ist die dem Siegel zugewandte und dem Innenraum abgewandte Seite der Austragsseite. Unter einer formschlüssigen Anordnung ist zu verstehen, dass das Siegel, insbesondere eine der Abdichtungsfläche gegenüberliegende Rückseitenfläche, und die Austragsseitenaußenfläche zumindest teilweise in direktem physischem Kontakt stehen. In dieser Ausgestaltungsform ist das Siegel sowohl mit der Austragsseitenöffnung, zur Befestigung des Siegels an der Vorrichtung, als auch mit der Austragsseitenaußenfläche in direktem physischem Kontakt.

Ein Vorteil des formschlüssigen Anliegens des Siegels an der Austragsseitenfläche ist eine hohe mechanische Belastbarkeit des Siegels beim Anpressen gegen den Knochenkanal. Das Anliegen an der Austragsseite sorgt für eine flächigere Kraftverteilung auf das Siegel beim Anpressen der Vorrichtung gegen den Knochenkanal, als dies bei nur punktueller Befestigung des Siegels, beispielsweise bei ausschließlichem physischem Kontakt mit der Austragsöffnung, der Fall wäre. Insbesondere wird durch das formschlüssige Anliegen die Verbindung zwischen Siegel und Vorrichtung mechanisch weniger beansprucht. Dadurch erhöht sich nicht nur die mechanische Belastbarkeit des Siegels, sondern auch die mechanische Belastbarkeit der Vorrichtung als Ganzes, insbesondere der Befestigungsstelle des Siegels, wie beispielsweise der Austragsöffnung. Vorteilhaft ist dies insbesondere bei Verkantungen des Siegels im Knochenkanal, welche aufgrund des hohen benötigten Anpressdrucks an den Knochen bei geringer mechanischer Stabilität zu Brüchen der Vorrichtung führen können. Die erhöhte mechanische Stabilität des Siegels und der Vorrichtung als Ganzes, erlaubt dem Operateur das Ausüben eines größeren Anpressdrucks an den Knochenkanal, was insbesondere bei nicht senkrechtem Anpresswinkel der Vorrichtung an den Knochen, ein vollständiges Abdichten der Knochenkanalöffnung erlaubt.

Eine weitere Ausgestaltungsform der Vorrichtung ist dadurch gekennzeichnet, dass der Applizierdruck ausreicht, um den Knochenzement mindestens 2 mm, insbesondere zwischen 3 bis 5 mm, tief in die Spongiosa eindringen zu lassen.

Eine geringere Eindringtiefe als 2 mm führt dazu, dass sich der Knochenzement, und die mit dem Knochenzement fixierte Prothese, insbesondere Hüftprothese, durch die Beanspruchungen des Gebrauchs, insbesondere bei Gehbewegungen des Patienten, lockert. Dies führt zu Folgeoperationen, welche bei einer Eindringtiefe über 2 mm vermieden, oder zumindest zeitlich herausgezögert werden können.

Sollte sich der Bereich der fixierten Prothese entweder im Anschluss an die Operation oder im Laufe der Zeit infizieren, muss die Prothese ausgewechselt werden. Dies erfolgt, unter anderem, durch die vollständige Entfernung des zur Fixierung verwendeten Knochenzements mitsamt des mit Knochenzements durchdrungenen Bereichs der Spongiosa. Um nicht übermäßig große Teile der Spongiosa zu Entfernen und auch bei der nachfolgenden Fixierung der neuen Prothese noch einen Teil der nicht entfernten Spongiosa wieder zur verbesserten Anhaftung von Knochen zu Knochenzement verwenden zu können, ist es bevorzugt, den Knochenzement nicht mehr als 5 mm tief in die Spongiosa eindringen zu lassen.

Eine weitere Ausgestaltungsform der Vorrichtung ist dadurch gekennzeichnet, dass das Siegel eine der Abdichtungsfläche gegenüberliegende Rückseitenfläche aufweist, wobei die Rückseitenfläche formschlüssig an der Austragsseitenaußenfläche angeordnet ist.

Der formschlüssige Kontakt zwischen der Rückseitenfläche und der Austragsseitenaußenfläche kann unterschiedlich ausgeformt sein. In einer Ausgestaltungsform sind zumindest 10 Flächen-% der Rückseitenfläche formschlüssig an der Austragsseitenaußenfläche angeordnet. Unter Flächen-% der Rückseitenfläche ist der Anteil der Rückseitenfläche zu verstehen, welcher, bezogen auf die Gesamtfläche der Rückseitenfläche, formschlüssig an der Austragsseitenfläche anliegt. Je höher dieser Anteil ist, desto größer ist die mechanische Belastbarkeit des Siegels, und damit auch die der Vorrichtung als Ganzes.

Eine weitere Ausgestaltungsform der Vorrichtung ist dadurch gekennzeichnet, dass die Rückseitenfläche mit zumindest 20 Flächen-% an der Austragsseitenaußenfläche angeordnet ist.

Die Rückseitenfläche kann mit bis zu 100 Flächen-% an der Austragsseitenaußenfläche angeordnet sein.

Die Rückseitenfläche, insbesondere der formschlüssig an die Austragsseitenaußenfläche angeordnete Teil der Rückseitenfläche, kann unterschiedlich ausgeformt sein. In einer Ausgestaltungsform ist die Rückseitenfläche planar ausgestaltet. In einer weiteren Ausgestaltungsform ist die gesamte Rückseitenfläche als Negativform der Austragsseitenaußenfläche ausgestaltet, so dass die gesamte Rückseitenfläche oder zumindest ein Teil der Rückseitenfläche formschlüssig an der Austragsseitenaußenfläche angeordnet ist.

Eine weitere Ausgestaltungsform der Vorrichtung ist dadurch gekennzeichnet, dass die Rückseitenfläche kreisringförmig an der Austragsseitenaußenfläche angeordnet ist.

Dazu weist die Rückseitenfläche eine kreisringförmige Struktur, beispielsweise ausgestaltet als eine Erhebung oder eine Vertiefung, auf, welche zumindest abschnittsweise als Negativform der Austragsseitenaußenfläche ausgeformt ist. Vorzugswiese befindet sich die kreisringförmige Struktur im Bereich der Ränder des Siegels, und damit auch im Bereich der durch die Außenwände des Behältnisses stabilisierten Ränder der Vorrichtung, so dass das Siegel insbesondere im Bereich der Siegelränder formschlüssig an der Vorrichtung anliegt, was eine erhöhte mechanische Belastbarkeit der Vorrichtung, insbesondere bei Verkantungen und nicht senkrechten Anpresswinkel der Vorrichtung an den Knochenkanal, sicherstellt. Das kreisringförmige Anliegen des Siegels an der Austragsseitenaußenfläche führt weiterhin zu einem möglichst breitgefächerten Anliegen über einen weiten Teil des Siegels und erhöht somit die mechanische Belastbarkeit des Siegels als auch der Vorrichtung als Ganzes. Vorzugsweise erstreckt sich die kreisringförmige Struktur der Rückseitenfläche nicht bis in den Bereich der Austragsöffnung, so dass die mechanische Belastung während des Anpressens an den Knochenkanal hauptsächlich auf die stabilen Ränder der Vorrichtung ausgeübt wird und nicht auf die weniger stabile Austragsöffnung.

Ein weiterer Gegenstand der Erfindung betrifft ein Verfahren zum Applizieren von Knochenzement in die Spongiosa eines Knochenkanals mittels einer Vorrichtung nach einer der vorherigen Ausführungsformen, zumindest umfassend die folgenden Schritte:
a) Bereitstellen des Knochenzements innerhalb der Vorrichtung,
b) Anpressen der Vorrichtung auf eine Knochenkanalöffnung, so dass der Knochenkanal während des Applizierens des Knochenzements durch ein Siegel abgedichtet ist,
c) Austragen des Knochenzements aus der Vorrichtung in den Knochenkanal,
wobei das Siegel im Wesentlichen auf eine Oberfläche des Knochens und/oder auf eine Schnittfläche des Knochens gepresst wird.

Das Bereitstellen des Knochenzements innerhalb der Vorrichtung, insbesondere innerhalb eines Behältnisses der Vorrichtung, in Schritt a) kann auf unterschiedliche Arten erfolgen. In einer Ausgestaltungsform des Verfahrens wird der Knochenzement außerhalb der Vorrichtung angemischt und danach in das Behältnis verbracht. In einer weiteren, bevorzugten Ausgestaltungsform des Verfahren, wird der Knochenzement innerhalb der Vorrichtung, insbesondere innerhalb des Behältnisses, angemischt, so dass der Anwender den Knochenzement nicht erst nach dem Anmischen umfüllen muss.

Vor dem Anpressen auf die Knochenkanalöffnung in Schritt b) wird die Vorrichtung mit einem Siegel ausgestattet. Dabei kann das Siegel zu unterschiedlichen Zeitpunkten an der Vorrichtung, insbesondere an einer Austragsöffnung der Vorrichtung, reversibel befestigt werden. In einer Ausgestaltungsform des Verfahrens, ist das Siegel bereits an der Vorrichtung befestigt, bevor der Knochenzement innerhalb des Behältnisses in Schritt a) bereitgestellt wird. In einer weiteren Ausgestaltungsform wird das Siegel erst nach dem Bereitstellen in Schritt a), aber vor dem Anpressen der Vorrichtung an die Knochenkanalöffnung in Schritt b), an der Vorrichtung befestigt.

Das Anpressen der Vorrichtung in Schritt c) erfolgt derart, dass kein Knochenzement aus dem Knochenkanal, insbesondere zwischen Siegel und Knochenkanalöffnung, austreten kann. Dadurch wird mit fortgesetztem Fördern von Knochenzement in den Knochenkanal ein ausreichender Applizierdruck aufgebaut, damit der Knochenzement zumindest teilweise in die Spongiosa des Knochens eingebracht wird. Dies sorgt für eine gute Anbindung von Knochenzement und Knochen und damit zu einer erhöhten mechanischen Belastbarkeit der fixierten Prothese, insbesondere Hüftprothese. Um einen ausreichend hohen Applizierdruck zum zumindest teilweisen Eindringen des Knochenzements in die Spongiosa zu erreichen, muss die Vorrichtung mit zumindest einem Anpressdruck gegen die Knochenkanalöffnung gepresst werden, welcher dem zu erreichenden Applizierdruck entspricht.

Das Verfahren ist dadurch gekennzeichnet, dass das Siegel in Schritt b) im Wesentlichen auf eine Oberfläche des Knochens und/oder auf eine Schnittfläche des Knochens gepresst wird. Dadurch ist das Siegel im Wesentlichen nicht in physischem Kontakt mit der Spongiosa, wodurch der Konchenzement freien Zugang zu der Spongiosa während des Applizierens erhält. Dies ermöglicht ein zumindest Einbringen des Knochenzements in die Spongiosa.

Ermöglicht wird das Anpressen im Wesentlichen auf eine Oberfläche des Knochens und/oder auf eine Schnittfläche des Knochens insbesondere durch ein Siegel mit einer konkaven Abdichtungsfläche, welches vorzugsweise wenigstens partiell formschlüssig an einer Austragsseitenaußenfläche der Vorrichtung angeordnet ist.

Eine Ausgestaltungsform des Verfahrens ist dadurch gekennzeichnet, dass in Schritt c) der Knochenkanal im Wesentlichen vollständig mit Knochenzement gefüllt wird. Ermöglicht wird dies insbesondere durch ein Siegel mit einer konkaven Abdichtungsfläche. Bei Siegeln, welche aufgrund einer nicht-konkaven Abdichtungsfläche tief in den Knochenkanal einreichen, muss nach erfolgter Applikation unter hohem Applizierdruck und nach Entfernen der Vorrichtung von der Knochenkanalöffnung, der durch das Siegel entstandene, nicht mit Knochenzement gefüllte, Hohlraum, in einem weiteren Applizierschritt, diesmal ohne Siegel, nachbefüllt werden. Bei im Wesentlichen nicht vollständig befüllten Knochenkanälen kann es bei der Fixierung einer Prothese zu Lufteinschlüssen zwischen Prothese und Knochenzement kommen, welche die mechanische Fixierung der Prothese verschlechtern.

Zudem kann der Knochenzement bei einer unvollständigen Befüllung des Knochenkanals nicht alle Bereiche der Spongiosa erreichen, was die Anhaftung von Knochenzement zu Knochen verringert.

Erfindungsgemäß wird unter einem Knochenzement eine Substanz verstanden, die geeignet ist im Bereich der Medizintechnik eine stabile Verbindung zwischen künstlichen Gelenken, wie beispielsweise Hüft- und Kniegelenken, und Knochengewebe zu erstellen. Bevorzugt handelt es sich bei Knochenzementen um Polymethylmethacrylat-Knochenzemente (PMMA Knochenzemente). PMMA-Knochenzemente kommen schon lange in medizinischen Anwendungen zum Einsatz und gehen auf Arbeiten von Sir Charnley zurück (vgl. Charnley, J. Anchorage of the femoral head prosthesis of the shaft of the femur. J. Bone Joint Surg. 1960; 42, 28-30.). PMMA-Knochenzemente können dabei aus einem Knochenzementpulver als erster Ausgangskomponente und einer Monomerflüssigkeit als zweiter Ausgangskomponente hergestellt werden. Bei geeigneter Zusammensetzung können die beiden Ausgangskomponenten getrennt voneinander lagerstabil sein. Bei Inkontaktbringen der beiden Ausgangskomponenten entsteht durch Quellung der Polymerbestandteile des Knochenzementpulvers ein plastisch verformbarer Knochenzement, der auch als Knochenzementteig bezeichnet wird. Dabei wird eine Polymerisation des Monomers durch Radikale eingeleitet. Mit fortschreitender Polymerisation des Monomers erhöht sich die Viskosität des Knochenzements, bis dieser vollständig aushärtet.

Erfindungsgemäß wird unter einem Knochenzementpulver ein Pulver verstanden, welches mindestens ein partikuläres Polymethylmethacrylat und/oder ein partikuläres Polymethylmethacrylat-Copolymer umfasst. Beispiele für Copolymere sind Styren und/oder Methylacrylat. In einer Ausgestaltungsform kann das Knochenzementpulvers zusätzlich ein hydrophiles Additiv umfassen, welches die Verteilung der Monomerflüssigkeit innerhalb des Knochenzementpulvers unterstützt. In einer weiteren Ausgestaltungsform kann das Knochenzementpulver zusätzlich einen Initiator, welcher die Polymerisation einleitet, umfassen. In einer weiteren Ausgestaltungsform kann das Knochenzementpulvers zusätzlich einen Röntgenopaker umfassen. In noch einer weiteren Ausgestaltungsform kann das Knochenzementpulver zusätzlich pharmazeutisch aktive Substanzen, wie beispielsweise Antibiotika, umfassen.

Bevorzugt umfasst das Knochenzementpulver mindestens ein partikuläres Polymethylmethacrylat und/oder ein partikuläres Polymethylmethacrylat-Copolymer, einen Initiator und einen Röntgenopaker oder besteht aus diesen Komponenten. Weiter bevorzugt umfasst das Knochenzementpulver mindestens ein partikuläres Polymethylmethacrylat und/oder ein partikuläres Polymethylmethacrylat-Copolymer, einen Initiator, einen Röntgenopaker und ein hydrophiles Additiv oder besteht aus diesen Komponenten. Am bevorzugtesten umfasst das Knochenzementpulver mindestens ein partikuläres Polymethylmethacrylat und/oder ein partikuläres Polymethylmethacrylat-Copolymer, einen Initiator, einen Röntgenopaker, ein hydrophiles Additiv und ein Antibiotikum oder besteht aus diesen Komponenten.

Erfindungsgemäß kann die Partikelgröße des partikulären Polymethylmethacrylat und/oder des partikulären Polymethylmethacrylat-Copolymers des Knochenzementpulvers der Siebfraktion kleiner 150 µm, bevorzugt kleiner 100 µm, entsprechen.

Erfindungsgemäß kann das hydrophile Additiv partikulär und/oder faserförmig ausgestaltet sein. In einer weiteren Ausgestaltungsform kann das hydrophile Additiv schwerlöslich, bevorzugt unlöslich, in Methylmethacrylat sein. In einer weiteren Ausgestaltungsform kann das hydrophile Additiv ein Aufsaugvermögen von mindestens 0,6 g Methylmethacrylat pro Gramm hydrophiles Additiv besitzen. In einer weiteren Ausgestaltungsform kann das hydrophile Additiv eine chemische Substanz mit mindestens einer OH-Gruppe aufweisen. Dabei kann bevorzugt vorgesehen sein, dass das hydrophile Additiv kovalent gebundene OH-Gruppen an seiner Oberfläche besitzt. Beispiele für solche bevorzugten hydrophilen Additive können Additive ausgewählt aus der Gruppe umfassend Cellulose, Oxycellulose, Stärke, Titandioxid und Siliziumdioxid sein, wobei pyrogenes Siliziumdioxid besonders bevorzugt ist. In einer Ausgestaltungsform kann die Partikelgröße des hydrophilen Additivs der Siebfraktion kleiner 100 µm, bevorzugt kleiner 50 µm und am bevorzugtesten kleiner 10 µm entsprechen. Das hydrophile Additiv kann in einer Menge von 0,1 bis 2,5 Gew.-% bezogen auf das Gesamtgewicht des Knochenzementpulvers enthalten sein.

Erfindungsgemäß kann der Initiator Dibenzoylperoxid enthalten oder aus Dibenzoylperoxid bestehen.

Erfindungsgemäß versteht man unter einem Röntgenopaker eine Substanz, welche es erlaubt, den Knochenzement auf röntgendiagnostischen Aufnahmen sichtbar zu machen. Beispiele für Röntgenopaker können Bariumsulfat, Zirkondioxid und Kalziumcarbonat umfassen. Erfindungsgemäß kann die pharmazeutisch aktive Substanz ein oder mehrere Antibiotika und gegebenenfalls zugesetzte Co-Faktoren für das eine oder die mehreren Antibiotika umfassen. Bevorzugt besteht die pharmazeutisch aktive Substanz aus einem oder mehreren Antibiotika und gegebenenfalls zugesetzten Co-Faktoren für das eine oder die mehreren Antibiotika. Beispiele für Antibiotika sind unter anderem Gentamicin, Clindamycin und Vancomycin. Erfindungsgemäß kann die Monomerflüssigkeit das Monomer Methylmethacrylat umfassen oder aus Methylmethacrylat bestehen. In einer Ausgestaltungsform umfasst die Monomerflüssigkeit neben dem Monomer einen darin gelösten Aktivator, wie beispielsweise N,N-Dimethyl-p-toluidin, oder besteht aus Methylmethacrylat und N,N-Dimethyl-p-toluidin.

### Beispiele

Die Erfindung wird im Folgenden durch Beispiele weiter beispielhaft illustriert. Die Erfindung ist nicht auf die Beispiele beschränkt.

### Figuren

Es zeigen
- Fig. 1: einen schematischen Querschnitt eines Siegels zum Abdichten eines Knochenkanals,
- Fig. 2: eine Aufsicht auf eine Abdichtungsfläche des Siegels aus Figur 1,
- Fig. 3: eine Aufsicht auf eine Rückseitenfläche des Siegels aus Figur 1,
- Fig. 4: einen schematischen Querschnitt einer Vorrichtung zum Applizieren von Knochenzement aufweisend das Siegel aus Figur 1, und
- Fig. 5: ein Verfahren zum Applizieren von Knochenzement in die Spongiosa eines Knochenkanals.

### Beschreibung der Figuren

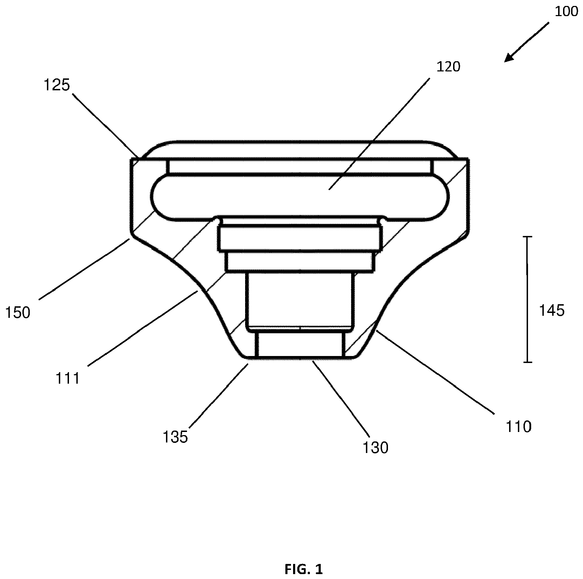

**Figur 1** zeigt ein Siegel 100 zum Abdichten eines Knochenkanals zur Verwendung im Zuge einer Applikation eines Knochenzement aus einer Vorrichtung. Um den Knochenzement aus der Vorrichtung durch das Siegel 100 in den Knochenkanal zu applizieren, weist das Siegel 100 einen Siegelkanal 120 auf. Der Siegelkanal 120 stellt eine fluidleitende, rohrartige Verbindung zwischen einer der beim Applizieren des Knochenzements dem Knochenkanal zugewandten Abdichtungsfläche 110 und einer der Abdichtungsfläche 110 entgegengesetzten und der Vorrichtung zugewandten Rückseitenfläche 125 dar, durch die der Knochenzement förderbar ist.

Die Abdichtungsfläche 110 weist eine Siegelöffnung 130 auf, in die der Siegelkanal 120 mündet und durch die der Knochenzement in den Knochenkanal förderbar ist. Die Siegelöffnung 130 ist von einem Siegelöffnungsbereich 135 umgeben, welcher sich radial um die Siegelöffnung 130 erstreckt. Während des Applizierens des Knochenzements in den Knochenkanal ragen die Siegelöffnung 130 und der Siegelöffnungsbereich 135 in den Knochenkanal hinein.

Das Siegel 100, insbesondere die Abdichtungsfläche 110, wird vor und während des Applizierens des Knochenzements gegen den Knochenkanal gepresst. Dies verhindert ein ungewolltes Austreten des Knochenzements aus dem Knochenkanal, wodurch mit fortgesetztem Applizieren des Knochenzements ein ausreichend hoher Applizierdruck erreicht wird, so dass der Knochenzement in eine Spongiosa des Knochenkanals zumindest teilweise eingebracht wird. Das Eindringen des Knochenzements in die Spongiosa erhöht die Haftkraft zwischen Knochen und Knochenzement und sorgt damit für eine verbesserte mechanische Fixierung einer Prothese, welche mittels des Knochenzements im Knochenkanal fixiert wird.

Die Abdichtungsfläche 110, welche den Siegelöffnungsbereich 135 umgibt, ist konkav ausgestaltet. Durch die konkave Ausgestaltung tritt eine Abdichtungsflächenmitte 111 gegenüber dem Siegelöffnungsbereich 135 und den Rändern 150 der Abdichtungsfläche 110 zurück. Die konkave Abdichtungsfläche 110 erlaubt eine gute Abdichtung eines Knochenkanals durch das Siegel 100, wobei die Abdichtungsfläche 110 aufgrund einer relativ geringen maximalen Bauhöhe 145 nur eine relativ geringe Eindringtiefe während des Applizierens in den Knochenkanal aufweisen. Ein Vorteil einer geringen Eindringtiefe ist, dass der Knochenkanal im Wesentlichen vollständig mit Knochenzement in nur einem Befüllvorgang befüllbar ist. Die konkave Abdichtungsfläche 110 erlaubt zudem ein zumindest teilweises Eindringen des Knochenzements in die Spongiosa des Knochenkanals, welche sich überwiegend im Bereich der Knochenkanalöffnung befindet.

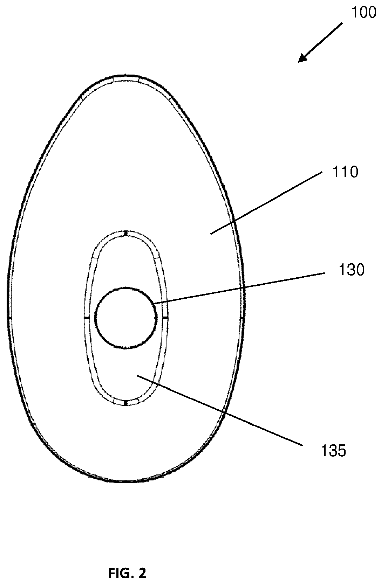

**Figur 2** zeigt das Siegel 100 aus Figur 1 in einer Aufsicht auf die Abdichtungsfläche 110. Die Siegelöffnung 130 ist umgeben vom Siegelöffnungsbereich 135. Die Siegelöffnung 130, und damit auch der Siegelöffnungsbereich 135, ist mittig in der Abdichtungsfläche 110 angeordnet. Der Siegelöffnungsbereich 135 und das gesamte Siegel 100 weisen eine ovale Querschnittsfläche auf. Ein Vorteil des ovalen Querschnitts ist, dass damit eine Vielzahl unterschiedlicher Knochenkanalgrößen und Knochenkanalformen abdichtbar sind.

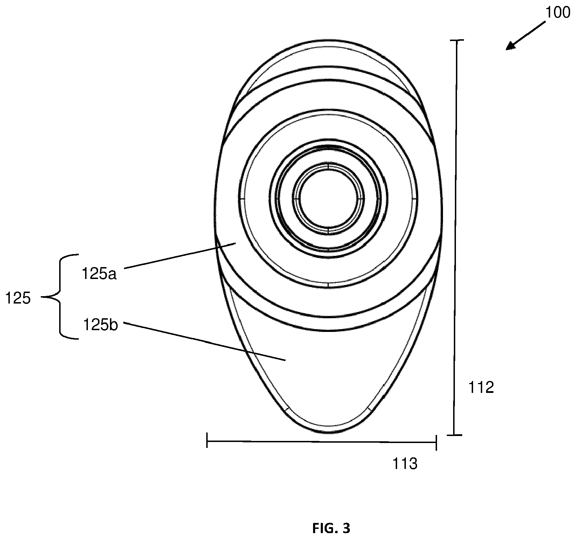

**Figur 3** zeigt das Siegel 100 aus den Figuren 1 und 2 in einer Aufsicht auf die Rückseitenfläche 125. Die Rückseitenfläche 125 weist einen kreisringförmigen Rückseitenflächenbereich A 125a und einen weiteren Rückseitenflächenbereich B 125b auf, wobei der Rückseitenflächenbereich A 125a dazu geeignet ist, formschlüssig an einer Vorrichtung zum Applizieren von Knochenzement angeordnet zu werden. Der kreisringförmige Rückseitenflächenbereich A 125a umfasst eine gesamte Breite 113 des ovalen Siegels 100, insbesondere der ovalen Rückseitenfläche 125. Der Rückseitenflächenbereich B 125b ist nicht dazu geeignet formschlüssig an der Vorrichtung angeordnet zu werden, sondern erstreckt sich radial, insbesondere entlang einer Länge 112 des Siegels 100, über einen Querschnitt der Vorrichtung hinaus. Dadurch wird dem Anwender des Siegels 100 die Handhabung, insbesondere ein Befestigen und Lösen des Siegels 100 an der Vorrichtung, erleichtert.

**Figur 4** zeigt eine Vorrichtung 200 zum Applizieren eines Knochenzements umfassend das Siegel 100 aus den Figuren 1 bis 3. Die Vorrichtung 200 weist ein Behältnis 300 auf, in dem ein Knochenzement lagerbar ist. An einem axialen Ende weist das Behältnis 300 eine Austragsseite 310 mit einer aus der Austragsseite 310 axial herausragenden Austragsöffnung 320 auf. Die Austragsöffnung 320 dient zum Austragen des Knochenzements aus dem Behältnis 300 in einen Knochenkanal. Die Austragsöffnung 320 ist in den Siegelkanal 120 eingebracht, wodurch das Siegel 100 form- und/oder kraftschlüssig an dem Behältnis 300 befestigt ist und gleichzeitig das Behältnis 300 fluidleitend für den Knochenzement mit der Siegelöffnung 130 verbunden ist. Die Verbindung zwischen der Austragsöffnung 320 und dem Siegel 100 ist derart ausgestaltet, dass der Knochenzement aus dem Behältnis 300 über die Austragsöffnung 320 und durch den Siegelkanal 120 appliziert werden kann, ohne dass es dabei zu einer Ablösung des Siegels 100 von der Austragsöffnung 320 kommt. In weiteren, nicht gezeigten Ausgestaltungsformen ist das Siegel 100 nicht durch einstecken der Austragsöffnung 320 in den Siegelkanal 120 mit dem Behältnis 300, sondern beispielsweise über eine Gewindeverbindung zwischen Austragsöffnung 320 und Siegelkanal 120 verbunden.

Das Siegel 100, insbesondere der kreisringförmige Rückseitenflächenbereich A 125a des Siegels 100 in Figur 3, ist formschlüssig an einer Austragsseitenaußenfläche 315 des Behältnisses 300 angeordnet. Da der kreisringförmige Rückseitenflächenbereich A 125a die gesamte Breite 113 des Siegels 100, insbesondere der ovalen Rückseitenfläche 125, umfasst, erhöht das formschlüssige Anliegen des Siegels 100 die mechanische Stabilität des Siegels 100 selbst sowie der gesamten Vorrichtung 200, insbesondere der Verbindung von Siegel 100 und Behältnis 300. Dies erlaubt ein Ausüben eines ausreichend hohen Anpressdrucks der Vorrichtung 200 an eine Knochenkanalöffnung um ein teilweises Einbringen des Knochenzements in die Spongiosa zu bewirken.

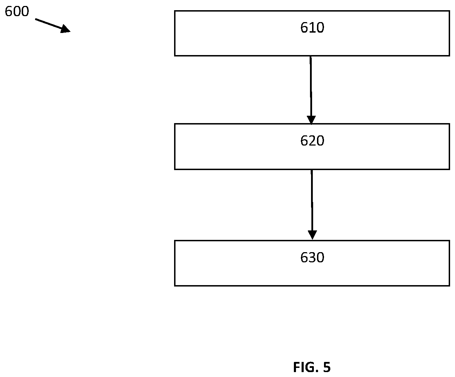

**Figur 5** zeigt ein Flussdiagramm enthaltend die Schritte 610 bis 630 eines Verfahrens 600 zum Applizieren eines Knochenzements in die Spongiosa eines Knochenkanals mittels der Vorrichtung 200 umfassend das Siegel 100. In einem Schritt 610 wird der Knochenzement innerhalb des Behältnisses 300 bereitgestellt. In einer Ausgestaltungsform des Verfahrens 600 wird der Knochenzement in dem Behältnis 300 bereitgestellt, indem ein zu Operationszwecken einsetzbarer, aus den entsprechenden Ausgangsstoffen gemischter Knochenzement in das Behältnis 300 eingebracht wird. In einer weiteren, bevorzugten, Ausgestaltungsform, wird der Knochenzement durch Mischen der entsprechenden Ausgangsstoffe innerhalb des Behältnisses 300 bereitgestellt. Letzteres ist bevorzugt, da der bereitgestellte Knochenzement nur für kurze Zeit, beispielweise in einem Zeitfenster von bis zu 5 Minuten nach dem Bereitstelen, verwendbar ist, bevor ein Aushärten zu weit fortgeschritten und das Applizieren somit nicht mehr möglich ist.

In einem Schritt 620 wird die Vorrichtung 200 auf eine Knochenkanalöffnung gepresst, so dass der Knochenkanal durch das Siegel 100 abgedichtet ist. Dabei kann es, aufgrund des relativ weichen Materials des Siegels, zu einer Verformung, insbesondere der Abdichtungsfläche 110, kommen. Durch die konkave Abdichtungsfläche 110 wird das Siegels 100 dabei im Wesentlichen auf eine Oberfläche des Knochens und/oder auf eine Schnittfläche des Knochens, welche in Vorbereitung der Operation durch den Operateur erzeugt wurde, und nicht auf eine Innenwand des Knochenkanals in Nähe der Knochenkanalöffnung, insbesondere nicht die Spongiosa des Knochenkanals, gepresst.

In einem Schritt 630 wird der Knochenzement aus der Vorrichtung 200 in den Knochenkanal, beispielsweise über einen im Behältnis 300 axial verschiebbaren Austragskolben, ausgetragen. Da das Siegel 100 während des Austragens im Wesentlichen auf die Oberseite des Knochens und/oder auf die Schnittfläche des Knochens gepresst wird und nicht gegen die Spongiosa des Knochenkanals, bleibt die Spongiosa im Zuge des Befüllens des Knochenkanals für den Knochenzement räumlich zugänglich. Durch das Abdichten des Knochenkanals mittels des Siegels 100 wird durch ein fortgeführtes Applizieren des Knochenzements in den Knochenkanal ein derart hoher Applizierdruck erreicht, dass der Knochenzement zumindest teilweise in die Spongiosa eingebracht wird. Dies erhöht die Haftung zwischen Knochen und Knochenzement und damit auch die mechanische Stabilität einer mittels des Knochenzements mit dem Knochen fixierten Prothese. Zudem dringt aufgrund der konkaven Abdichtungsfläche 110 das Siegel 100 nur so weit in den Knochenkanal ein, dass ein im Wesentlichen vollständiges Befüllen des Knochenkanals mit dem Knochenzement in Schritt 630 durchgeführt werden kann, wodurch kein zusätzlicher Befüllschritt notwendig wird, bevor die zu fixierende Prothese in den gefüllten Knochenkanal eingesetzt wird. Dies ist gerade im Zuge zeitkritischer Operationen von Vorteil und sorgt zudem für eine homogenere Beschaffenheit des Knochenzements, welche sich in einer höheren mechanischen Stabilität des Knochenzements widerspiegelt.

Die in den Ansprüchen, der Beschreibung und in den Figuren offenbarten Merkmale können für verschiedene Ausgestaltungsformen der beanspruchten Erfindung sowohl getrennt als auch in beliebiger Kombination miteinander wesentlich sein. Die für das Siegel und die Vorrichtung offenbarten Merkmale sind auch für das Verfahren offenbart und umgekehrt.

### Bezugszeichen

- 100: Siegel
- 110: Abdichtungsfläche
- 111: Abdichtungsflächenmitte
- 112: Länge
- 113: Breite
- 120: Siegelkanal
- 125: Rückseitenfläche
- 125a: Rückseitenflächenbereich A
- 125b: Rückseitenflächenbereich B
- 130: Siegelöffnung
- 135: Siegelöffnungsbereich
- 145: maximale Bauhöhe
- 150: Ränder
- 200: Vorrichtung
- 300: Behältnis
- 310: Austragsseite
- 315: Austragsseitenaußenfläche
- 320: Austragsöffnung
- 600: Verfahren zum Applizieren von Knochenzement
- 610: Bereitstellen des Knochenzements
- 620: Anpressen der Vorrichtung
- 630: Austragen des Knochenzements

## Patentansprüche

1. Siegel (100) zum Abdichten einer Knochenkanalöffnung beim Applizieren von Knochenzement in die Spongiosa eines Knochenkanals,
**dadurch gekennzeichnet, dass**
das Siegel (100) eine konkave Abdichtungsfläche (110) zum Abdichten des Knochenkanals aufweist.

2. Siegel (100) nach Anspruch 1, **dadurch gekennzeichnet, dass** das Siegel (100) einen Siegelkanal (120) aufweist, wobei der Siegelkanal (120) reversibel form- und/oder kraftschlüssig mit einer Austragsöffnung (320) einer den Knochenzement bereitstellenden Vorrichtung (200) verbindbar ist, um den Knochenzement aus einer Siegelöffnung (130) an einem der Abdichtungsfläche (110) zugewandten Ende des Siegelkanals (120) aus der Vorrichtung (200) in den Knochenkanal zu applizieren.

3. Siegel (100) nach Anspruch 1 oder 2, **dadurch gekennzeichnet, dass** die Siegelöffnung (130) mittig in der Abdichtungsfläche (110) angeordnet ist.

4. Siegel (100) nach Anspruch 2 oder 3, **dadurch gekennzeichnet, dass** die Abdichtungsfläche (110) eine Bauhöhe aufweist, wobei eine maximale Bauhöhe (145) des Siegels (100) angrenzend an einen Siegelöffnungsbereich (135) vorliegt und die Bauhöhe vom Siegelöffnungsbereich (135) in Richtung der Ränder (150) der Abdichtungsfläche (110) mit einer konkaven Funktion abnimmt.

5. Siegel (100) nach Anspruch 4, **dadurch gekennzeichnet, dass** die konkave Funktion angrenzend an den Siegelöffnungsbereich (135) eine größte Steigung aufweist und die Steigung in Richtung der Ränder (150) der Abdichtungsfläche (110) stetig abnimmt.

6. Siegel (100) nach Anspruch 4 oder 5, **dadurch gekennzeichnet, dass** die maximale Bauhöhe (145) der Abdichtungsfläche (110) im Bereich von 5 bis 20 mm liegt.

7. Siegel (100) nach einem der vorhergehenden Ansprüche, **dadurch gekennzeichnet, dass** die Abdichtungsfläche (110) eine ovale Querschnittsfläche mit einer Länge (112) von im Bereich von 60 bis 80 mm und einer Breite (113) im Bereich von 25 bis 45 mm aufweist.

8. Siegel (100) nach einem der vorhergehenden Ansprüche, **dadurch gekennzeichnet, dass** das Siegel einen Kunststoff, insbesondere einen thermoplastischen Kunststoff, mit einer Shore-Härte von 25 bis 50 ShoreA umfasst, bevorzugt aus einem Kunststoff, insbesondere aus einem thermoplastischen Kunststoff, mit einer Shore-Härte von 25 bis 50 ShoreA besteht.

9. Vorrichtung (200) zum Applizieren von Knochenzement in die Spongiosa eines Knochenkanals,
umfassend
ein Behältnis (300), in dem ein Knochenzement lagerbar ist,
wobei das Behältnis (300) an einem Ende eine Austragsseite (310) mit einer aus der Austragsseite (310) herausragenden Austragsöffnung (320) zum Austragen des Knochenzements aus dem Behältnis (200) in den Knochenkanal aufweist,
**dadurch gekennzeichnet, dass**
die Austragsöffnung (320) reversibel form- und/oder kraftschlüssig mit einem Siegel (100) nach einem der Ansprüche 1 bis 8 zum Abdichten einer Knochenkanalöffnung verbunden ist, um den Knochenzement unter ausreichendem Applizierdruck in den Knochenkanal zu applizieren.

10. Vorrichtung (200) nach Anspruch 9, **dadurch gekennzeichnet, dass** der Applizierdruck ausreicht, um den Knochenzement mindestens 2 mm, insbesondere zwischen 3 bis 5 mm, tief in die Spongiosa eindringen zu lassen.

11. Vorrichtung (200) nach Anspruch 9 oder 10, **dadurch gekennzeichnet, dass** das Siegel (100) wenigstens partiell formschlüssig an einer Austragsseitenaußenfläche (315) angeordnet ist.

12. Vorrichtung (200) nach Anspruch 9 bis 11, **dadurch gekennzeichnet, dass** das Siegel (100) eine der Abdichtungsfläche (110) gegenüberliegende Rückseitenfläche (125) aufweist, wobei die Rückseitenfläche (125) formschlüssig an der Austragsseitenaußenfläche (315) angeordnet ist.

13. Vorrichtung (200) nach Anspruch 12, **dadurch gekennzeichnet, dass** die Rückseitenfläche (125) mit zumindest 20 Flächen-% an der Austragsseitenaußenfläche (315) angeordnet ist.

14. Vorrichtung (200) nach Anspruch 12 oder 13, **dadurch gekennzeichnet, dass** die Rückseitenfläche (125) kreisringförmig an der Austragsseitenaußenfläche (315) angeordnet ist.

15. Verfahren (600) zum Applizieren von Knochenzement in die Spongiosa eines Knochenkanals mittels einer Vorrichtung (200) nach einem der Ansprüche 9 bis 14, **zumindest umfassend die folgenden Schritte:**
a) Bereitstellen des Knochenzements innerhalb der Vorrichtung (200),
b) Anpressen der Vorrichtung (200) auf eine Knochenkanalöffnung, so dass der Knochenkanal während des Applizierens des Knochenzements durch ein Siegel (100) abgedichtet ist,
c) Austragen des Knochenzements aus der Vorrichtung (200) in den Knochenkanal,
wobei das Siegel (100) im Wesentlichen auf eine Oberfläche des Knochens und/oder auf eine Schnittfläche des Knochens gepresst wird.
